# EUROPÄISCHE PATENTSCHRIFT

(11) **EP 3 802 497 B1**
(45) Veröffentlichungstag und Bekanntmachung des Hinweises auf die Patenterteilung: **06.07.2022**
(21) Anmeldenummer: 19727946.6
(22) Anmeldetag: 20.05.2019
(51) Int. Cl.: C07D 231/16

(54) **VERFAHREN ZUR HERSTELLUNG VON HALOGENIERTEN N-ARYLPYRAZOLEN**
METHOD FOR THE PREPARATION OF HALOGENATED N-ARYLPYRAZOLES
PROCÉDÉ DE PRODUCTION DE N-ARYLPYRAZOLES HALOGÉNÉS

(30) Priorität: 24.05.2018 EP 18174030
(43) Veröffentlichungstag der Anmeldung: 14.04.2021
(73) Patentinhaber: Bayer Animal Health GmbH, 51373 Leverkusen (DE)
(72) Erfinder: REMBIAK, Andreas, 65812 Bad Soden (DE); HEILMANN, Eike Kevin, 40599 Düsseldorf (DE)
(74) Vertreter: Cohausz & Florack
(86) Internationale Anmeldenummer: PCT/EP2019/062924
(87) Internationale Veröffentlichungsnummer: WO 2019/224139

(56) Entgegenhaltungen:
- WO-A1-2015/067647
- REBECCA T. RUCK ET AL: "Route Development and Multikilogram GMP Delivery of a Somatostatin Receptor Antagonist", ORGANIC PROCESS RESEARCH AND DEVELOPMENT, Bd. 16, Nr. 8, 1. August 2012 (2012-08-01) , Seiten 1329-1337, XP055489425, US ISSN: 1083-6160, DOI: 10.1021/op300128c in der Anmeldung erwähnt
- CASTANET ANNE-SOPHIE ET AL: "Mild and regioselective iodination of electron-rich aromatics withN-iodosuccinimide and catalytic trifluoroacetic acid", TETRAHEDRON LETTERS, Bd. 43, Nr. 29, 2002, Seiten 5047-5048, XP085047492, ISSN: 0040-4039, DOI: 10.1016/S0040-4039(02)01010-9 in der Anmeldung erwähnt

## Beschreibung

Die vorliegende Erfindung betrifft ein Verfahren zur Herstellung von Verbindungen der Formel (I) 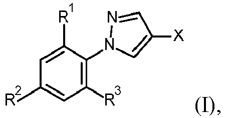 durch Halogenierung von Verbindungen der Formel (II) 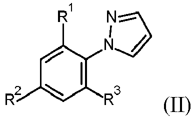 worin R¹, R², R³ und X wie nachfolgend definiert sind.

Verbindungen gemäß Formel (I) und deren Herstellung sind beispielsweise in WO2015/067646, WO2015/067647, WO2016/174052 und in WO2017/025590 beschrieben. Nachteilig an den darin beschriebenen Herstellungsverfahren sind jedoch die hohen Reaktionstemperaturen, die zum Teil langen Reaktionszeiten sowie die starken Schwankungen bei den erhaltenen Ausbeuten an Verbindungen der allgemeinen Formel (I).

Alternative, allgemein in der Literatur bekannte Möglichkeiten zur Halogenierung von Pyrazolen beschreiben die Verwendung von elementarem Iod oder Brom (WO 2008/156739) sowie anorganischen Iod- und Brom-Salzen *(*Russ. Chem. Bull. 2014, 63, 360, RSC Advances 2016, 6, 90031), gegebenenfalls unter Zusatz oxidierender Verbindungen wie Wasserstoffperoxid *(*Tetrahedron Lett. 2008, 49, 4026) oder Cerammoniumnitrat (US 2015/322063, US 2011/166143). Nachteilig bei diesen Verfahren sind die Notwendigkeit erhöhter Temperaturen, zum Teil unvollständige oder nur geringe Umsätze zu Verbindungen der allgemeinen Formel (I), die aufwendige Abtrennung von Schwermetallsalzen sowie die Freisetzung giftiger und korrosiver Brom- oder Ioddämpfe. Aus diesen Gründen sind diese Verfahren für eine technische Anwendung nicht geeignet.

Des Weiteren ist die Aktivierung organischer, Iod-haltiger Moleküle zur Iodierung durch den Einsatz starker Säuren in der Literatur beschrieben (Tetrahedron Lett. 2002, 43, 5047; Tetrahedron Lett. 2009, 50, 2664; Org. Proc. Res. Dev. 2012, 16, 1329). Nachteilig bei diesen Verfahren ist die Beschränkung auf aktivierte Pyrazole und Aromaten im Allgemeinen sowie die Verwendung größerer katalytischer oder stöchiometrischer Mengen starker Säuren.

Halogenierte *N*-Arylpyrazol-Derivate haben jedoch eine große Bedeutung als Baustein zur Synthese neuer agrochemischer Wirkstoffe. Daher bestand die Aufgabe der vorliegenden Erfindung darin, ein Verfahren zur Herstellung von Verbindungen der allgemeinen Formel (I) bereitzustellen, das großtechnisch und kostengünstig eingesetzt werden kann und die oben beschriebenen Nachteile umgeht. Auch ist es erstrebenswert, die speziellen *N*-Arylpyrazol-Derivate mit hoher Ausbeute und hoher Reinheit zu erhalten, so dass die Zielverbindung vorzugsweise keiner weiteren eventuell aufwendigen Aufreinigung unterzogen werden muss.

Diese Aufgabe wurde erfindungsgemäß gelöst durch ein Verfahren zur Herstellung von Verbindungen der Formel (I) 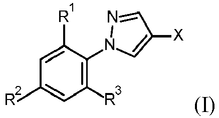 worin
- X: für Halogen steht;
- R¹: für Wasserstoff, Cyano, Halogen, gegebenenfalls mit Halogen oder CN substituiertes C₁-C₄-Alkyl oder für gegebenenfalls mit Halogen substituiertes C₁-C₄-Alkoxy steht,
- R²: für Halogen, Trifluormethylsulfonyl, Trifluormethylsulfinyl, Trifluormethylsulfanyl, gegebenenfalls mit Halogen substituiertes C₁-C₄-Alkyl oder für gegebenenfalls mit Halogen substituiertes C₁-C₄-Alkoxy steht und
- R³: für Wasserstoff, Cyano, Halogen, gegebenenfalls mit Halogen oder CN substituiertes C₁-C₄-Alkyl oder für gegebenenfalls mit Halogen substituiertes C₁-C₄-Alkoxy steht,
durch Halogenierung von Verbindungen der Formel (II) 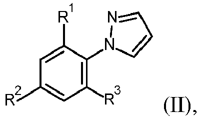 worin R¹, R² und R³ wie oben stehend definiert sind,
mit einer organischen halogenierenden Verbindung unter Zusatz von ≥ 0,0001 Äquivalenten und < 0,3 Äquivalenten, bezogen auf die gesamte eingesetzte Stoffmenge an Verbindung der Formel (II), wenigstens einer Säure, ausgewählt aus Mineralsäuren, Sulfonsäuren, Carbonsäuren und Lewis-Säuren.

Überraschend wurde nun gefunden, dass das erfindungsgemäße Verfahren durch den Zusatz von geringen katalytischen Mengen einer Säure bereits bei niedrigen Temperaturen zu einer schnellen Halogenierung mit konstanter, sehr guter Ausbeute an Verbindungen der allgemeinen Formel (I) führt. Zudem ermöglicht das erfindungsgemäße Verfahren eine Dosier-kontrollierte Reaktionsführung und führt zugleich zu einer Verbesserung der Prozesssicherheit.

Die im Folgenden beschriebenen bevorzugten Ausführungsformen beziehen sich, wenn zutreffend, auf alle hierin beschriebenen Formeln.

In einer bevorzugten Ausführungsform der Erfindung steht
- R²: für mit Halogen substituiertes C₁-C₄-Alkyl oder mit Halogen substituiertes C₁-C₄-Alkoxy, wie beispielsweise Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl, Heptafluor-n-propyl, Heptafluor-isopropyl, Nonafluor-n-butyl, Nonafluor-sec-butyl, Nonafluor-tert-butyl, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy oder Pentafluorethoxy.

Besonders bevorzugt steht
- R²: für mit Fluor substituiertes C₁-C₄-Alkyl oder mit Fluor substituiertes C₁-C₄-Alkoxy.

Ganz besonders bevorzugt steht
- R²: für Perfluoro- C₁-C₃-Alkyl (CF₃, C₂F₅ oder C₃F₇ (n- oder iso-Propyl)) oder Perfluoro- C₁-C₃-Alkoxy (OCF₃, OC₂F₅ oder OC₃F₇ (n- oder iso-Propoxy)).

Insbesondere bevorzugt steht
- R²: für Perfluoro- C₁-C₃-Alkyl, wie Trifluormethyl, Pentafluorethyl, Heptafluor-iso-propyl oder Heptafluor-n-propyl, insbesondere für Heptafluor-iso-propyl.

In einer weiteren bevorzugten Ausführungsform stehen R¹ und R³ jeweils unabhängig voneinander für einen Substituenten ausgewählt aus Wasserstoff, Cl, Br, F, C₁-C₃-alkyl, mit Halogen substituiertes C₁-C₃-alkyl, C₁-C₃-alkoxy oder mit Halogen substituiertes C₁-C₃-alkoxy.

In einer weiteren bevorzugten Ausführungsform stehen R¹ und R³ für die hierin beschriebenen Substituenten, jedoch stehen R¹ und R³ nicht gleichzeitig in einer Verbindung für Wasserstoff.

Mit anderen Worten, wenn R¹ in einer Verbindung für Wasserstoff steht, steht R³ für einen der anderen hierin beschriebenen Substituenten und umgekehrt.

In einer besonders bevorzugten Ausführungsform stehen R¹ und R³ jeweils unabhängig voneinander für Cl, Br, C₁-C₃-alkyl, oder mit Fluor substituiertes C₁-C₃-alkyl, C₁-C₃-alkoxy oder mit Fluor substituiertes C₁-C₃-alkoxy, insbesondere für Cl, Br, Methyl, Trifluormethyl, Trifluormethoxy oder Difluormethoxy.

In einer ganz besonders bevorzugten Ausführungsform steht R¹ und R³ unabhängig voneinander für Cl, Br oder F, insbesondere für Cl oder Br. In einer besonders vorteilhaften Ausgestaltung der Erfindung stehen R¹ und R³ für dasselbe Halogen, insbesondere für Chlor.

In einer bevorzugten Ausgestaltung der Erfindung steht wenigstens einer der Reste R¹, R², R³ für mit Halogen substituiertes C₁-C₄-Alkyl oder für mit Halogen substituiertes C₁-C₄-Alkoxy, besonders bevorzugt für mit Fluor substituiertes C₁-C₃-Alkyl oder für mit Fluor substituiertes C₁-C₃-Alkoxy .

In einer weiteren besonders vorteilhaften Ausgestaltung der Erfindung steht
R¹ für Halogen oder C₁-C₃-alkyl, insbesondere für Br, Cl oder Methyl,
R² für mit Fluor substituiertes C₁-C₄-Alkyl oder mit Fluor substituiertes C₁-C₄-Alkoxy, insbesondere Heptafluor-iso-propyl und
R³ für Halogen, C₁-C₃-alkyl oder mit Fluor substituiertes C₁-C₃-alkyl, C₁-C₃-alkoxy oder mit Fluor substituiertes C₁-C₃-alkoxy, insbesondere Cl, Methyl, Trifluormethyl, Trifluormethoxy oder Difluormethoxy.

In einer bevorzugten Ausgestaltung der Erfindung steht X für Chlor, Brom oder Iod, besonders bevorzugt für Brom oder Iod und ganz besonders bevorzugt für Iod.

Die als Ausgangsprodukte verwendeten Pyrazole der Formel (II) können beispielsweise in Analogie zu der in WO2015/067646, WO2015/067647 und WO2016/174052 beschriebenen Methode aus den entsprechenden Hydrazin-Derivaten hergestellt werden.

Bevorzugt eingesetzte Pyrazole der Formel (II) sind
1- [4-(1,1,1,2,3,3,3-Heptafluorpropan-2-yl)-2,6-dimethylphenyl]-1H-pyrazol
1-[2,6-Dichlor-4-(1,1,1,2,3,3,3-heptafluorpropan-2-yl)phenyl]-1H-pyrazol
1-[2-Chlor-4-(1,1,1,2,3,3,3-heptafluorpropan-2-yl)-6-(trifluormethyl)phenyl]-1H-pyrazol
1-[2-Chlor-4-(1,1,1,2,3,3,3-heptafluorpropan-2-yl)-6-(trifluormethoxy)phenyl]-1H-pyrazol
1-[2-Chlor-6-(difluormethoxy)-4-(1,1,1 ,2,3,3,3-heptafluorpropan-2-yl)phenyl] -1H-pyrazol
1-[4-(1,1,1,2,3,3,3-Heptafluorpropan-2-yl)-2-methyl-6-(trifluormethyl)phenyl]-1H-pyrazol
1-[2-Brom-4-(1,1,1,2,3,3,3-heptafluorpropan-2-yl)-6-(trifluormethyl)phenyl]-1H-pyrazol
1-[2-Brom-4-(1,1,1,2,3,3,3-heptafluorpropan-2-yl)-6-(trifluormethoxy)phenyl]-1H-pyrazol

Besonders bevorzugt sind dabei:
1-[2,6-Dichlor-4-(1,1,1,2,3,3,3-heptafluorpropan-2-yl)phenyl]-1H-pyrazol
1-[2-Chlor-4-(1,1,1,2,3,3,3-heptafluorpropan-2-yl)-6-(trifluormethyl)phenyl]-1H-pyrazol
1-[2-Chlor-4-(1,1,1,2,3,3,3-heptafluorpropan-2-yl)-6-(trifluormethoxy)phenyl]-1H-pyrazol
1-[2-Chlor-6-(difluormethoxy)-4-(1,1,1 ,2,3,3,3-heptafluorpropan-2-yl)phenyl]-1H-pyrazol
1-[2-Brom-4-(1,1,1,2,3,3,3-heptafluorpropan-2-yl)-6-(trifluormethyl)phenyl]-1H-pyrazol
1-[2-Brom-4-(1,1,1,2,3,3,3-heptafluorpropan-2-yl)-6-(trifluormethoxy)phenyl]-1H-pyrazol
Ganz besonders bevorzugt ist 1-[2,6-Dichlor-4-(1,1,1,2,3,3,3-heptafluorpropan-2-yl)phenyl]-1H-pyrazol.

Aus diesen Verbindungen ergeben sich entsprechend die folgenden bevorzugten Verbindungen der Formel (I):
4-Brom-1-[4-(1,1,1,2,3,3,3-heptafluorpropan-2-yl)-2,6-dimethylphenyl]-1H-pyrazol
4-Brom-1-[2,6-dichlor-4-(1,1,1,2,3,3,3-heptafluorpropan-2-yl)phenyl]-1H-pyrazol
4-Brom-1-[2-chlor-4-(1,1,1,2,3,3,3-heptafluorpropan-2-yl)-6-(trifluormethyl)phenyl]-1H-pyrazol
4-Brom-1-[2-chlor-4-(1,1,1,2,3,3,3-heptafluorpropan-2-yl)-6-(trifluormethoxy)phenyl]-1H-pyrazol
4-Brom-1-[2-chlor-6-(difluormethoxy)-4-(1,1,1,2,3,3,3-heptafluorpropan-2-yl)phenyl]-1H-pyrazol
4-Brom-1-[4-(1,1,1,2,3,3,3-heptafluorpropan-2-yl)-2-methyl-6-(trifluormethyl)phenyl]-1H-pyrazol
4-Brom-1-[2-brom-4-(1,1,1,2,3,3,3-heptafluorpropan-2-yl)-6-(trifluormethyl)phenyl]-1H-pyrazol
4-Brom-1-[2-brom-4-(1,1,1,2,3,3,3-heptafluorpropan-2-yl)-6-(trifluormethoxy)phenyl] -1H-pyrazol
1-[4-(1,1,1,2,3,3,3-Heptafluorpropan-2-yl)-2,6-dimethylphenyl]-4-iod-1H-pyrazol
1-[2,6-Dichlor-4-(1,1,1,2,3,3,3-heptafluorpropan-2-yl)phenyl]-4-iod-1H-pyrazol
1-[2-Chlor-4-(1,1,1,2,3,3,3-heptafluorpropan-2-yl)-6-(trifluormethyl)phenyl]-4-iod-1H-pyrazol
1-[2-Chlor-4-(1,1,1,2,3,3,3-heptafluorpropan-2-yl)-6-(trifluormethoxy)phenyl]-4-iod-1H-pyrazol
1-[2-Chlor-6-(difluormethoxy)-4-(1,1,1,2,3,3,3-heptafluorpropan-2-yl)phenyl]-4-iod-1H-pyrazol
1-[4-(1,1,1,2,3,3,3-Heptafluorpropan-2-yl)-2-methyl-6-(trifluormethyl)phenyl]-4-iod-1H-pyrazol
1-[2-Brom-4-(1,1,1,2,3,3,3-heptafluorpropan-2-yl)-6-(trifluormethyl)phenyl]-4-iod-1H-pyrazol
1-[2-Brom-4-(1,1,1 ,2,3,3,3-heptafluorpropan-2-yl)-6-(trifluormethoxy)phenyl]-4-iod-1H-pyrazol

Besonders bevorzugt sind dabei
4-Brom-1-[2,6-dichlor-4-(1,1,1,2,3,3,3-heptafluorpropan-2-yl)phenyl]-1H-pyrazol
4-Brom-1-[2-chlor-4-(1,1,1,2,3,3,3-heptafluorpropan-2-yl)-6-(trifluormethyl)phenyl]-1H-pyrazol
4-Brom-1-[2-chlor-4-(1,1,1,2,3,3,3-heptafluorpropan-2-yl)-6-(trifluormethoxy)phenyl]-1H-pyrazol
4-Brom-1-[2-chlor-6-(difluormethoxy)-4-(1,1,1,2,3,3,3-heptafluorpropan-2-yl)phenyl]-1H-pyrazol
4-Brom-1-[4-(1,1,1,2,3,3,3-heptafluorpropan-2-yl)-2-methyl-6-(trifluormethyl)phenyl]-1H-pyrazol
4-Brom-1-[2-brom-4-(1,1,1,2,3,3,3-heptafluorpropan-2-yl)-6-(trifluormethyl)phenyl]-1H-pyrazol
4-Brom-1-[2-brom-4-(1,1,1,2,3,3,3-heptafluorpropan-2-yl)-6-(trifluormethoxy)phenyl]-1H-pyrazol
1-[2,6-Dichlor-4-(1,1,1,2,3,3,3-heptafluorpropan-2-yl)phenyl]-4-iod-1H-pyrazol
1-[2-Chlor-4-(1,1,1,2,3,3,3-heptafluorpropan-2-yl)-6-(trifluormethyl)phenyl]-4-iod-1H-pyrazol
1-[2-Chlor-4-(1,1,1,2,3,3,3-heptafluorpropan-2-yl)-6-(trifluormethoxy)phenyl]-4-iod-1H-pyrazol
1-[2-Chlor-6-(difluormethoxy)-4-(1,1,1,2,3,3,3-heptafluorpropan-2-yl)phenyl]-4-iod-1H-pyrazol
1-[4-(1,1,1,2,3,3,3-Heptafluorpropan-2-yl)-2-methyl-6-(trifluormethyl)phenyl]-4-iod-1H-pyrazol
1-[2-Brom-4-(1,1,1,2,3,3,3-heptafluorpropan-2-yl)-6-(trifluormethyl)phenyl]-4-iod-1H-pyrazol
1-[2-Brom-4-(1,1,1 ,2,3,3,3-heptafluorpropan-2-yl)-6-(trifluormethoxy)phenyl]-4-iod-1H-pyrazol

Ganz besonders bevorzugt sind
1-[2,6-Dichlor-4-(1,1,1,2,3,3,3-heptafluorpropan-2-yl)phenyl]-4-iod-1H-pyrazol,
1-[2-Chlor-4-(1,1,1,2,3,3,3-heptafluorpropan-2-yl)-6-(trifluormethyl)phenyl]-4-iod-1H-pyrazol,
1-[2-Chlor-4-(1,1,1,2,3,3,3-heptafluorpropan-2-yl)-6-(trifluormethoxy)phenyl]-4-iod-1H-pyrazol und
1-[2-Brom-4-(1,1,1,2,3,3,3-heptafluorpropan-2-yl)-6-(trifluormethoxy)phenyl]-4-iod-1H-pyrazol.

Sofern nicht an anderer Stelle anders definiert, wird unter dem Begriff "Alkyl", erfindungsgemäß entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen, bevorzugt mit 1 bis 6, besonders bevorzugt mit 1 bis 4 Kohlenstoffatomen, verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Sofern nicht an anderer Stelle anders definiert, wird unter dem Begriff "Aryl" erfindungsgemäß ein aromatischer Rest mit 6 bis 14 Kohlenstoffatomen, vorzugsweise Phenyl, Naphthyl, Anthryl oder Phenanthrenyl, besonders bevorzugt Phenyl, verstanden.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Bereiche gelten für das Gesamtverfahren entsprechend. Diese Definitionen können untereinander, also auch zwischen den jeweiligen Vorzugsbereichen, beliebig kombiniert werden.

Erfindungsgemäß bevorzugt verwendet werden Verfahren, in welchen eine Kombination der vorstehend als bevorzugt aufgeführten Bedeutungen und Bereiche vorliegt.

Erfindungsgemäß besonders bevorzugt verwendet werden Verfahren, in welchen eine Kombination der vorstehend als besonders bevorzugt aufgeführten Bedeutungen und Bereiche vorliegt.

Erfindungsgemäß ganz besonders bevorzugt verwendet werden Verfahren, in welchen eine Kombination der vorstehend als ganz besonders bevorzugt aufgeführten Bedeutungen und Bereiche vorliegt.

Erfindungsgemäß insbesondere verwendet werden Verfahren, in welchen eine Kombination der vorstehend als insbesondere aufgeführten Bedeutungen und Bereiche vorliegt.

Erfindungsgemäß hervorgehoben verwendet werden Verfahren, in welchen eine Kombination der vorstehend als hervorgehoben aufgeführten Bedeutungen und Bereiche vorliegt.

### Verfahrensbeschreibung

### Herstellung von Verbindungen der Formel (I)

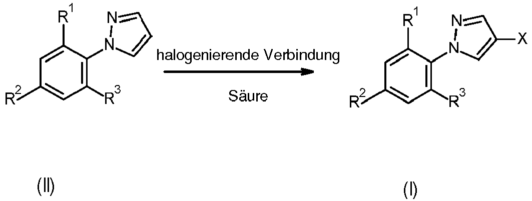

Die Reste X, R¹ R² und R³ haben die oben beschriebenen Bedeutungen. Die Verbindungen der Strukturformeln (I) und (II) sind z.B. die oben als bevorzugte Pyrazole und Halogenpyrazole genannten Verbindungen.

Die Halogenpyrazole der allgemeinen Formel (I) werden mit dem erfindungsgemäßen Verfahren mit guten Ausbeuten und in hoher Reinheit erhalten.

Erfindungsgemäße Verbindungen der allgemeinen Struktur (I) werden hergestellt indem man die Pyrazole der Struktur (II) mit halogenierenden Verbindungen unter Zusatz von ≥ 0,0001 Äquivalenten und < 0,3 Äquivalenten bezogen auf die gesamte eingesetzte Stoffmenge an Verbindung (II), wenigstens einer Säure umsetzt.

Geeignete organische halogenierende Verbindungen sind dabei bevorzugt ausgewählt aus den *N*-Halogensuccinimiden, insbesondere aus *N*-Chlorsuccinimid (NCS), *N*-Bromsuccinimid (NBS) oder *N*-Iodsuccinimid (NIS), den 1,3-Dihalogen-5,5-dimethylhydantoinen, insbesondere aus 1,3-Chlor-5,5-dimethylhydantoin (DCDMH), 1,3-Dibrom-5,5-dimethylhydantoin (DBDMH) oder 1,3-Diiod-5,5-dimethylhydantoin (DIDMH) oder den Halogencyanursäuren, insbesondere aus 1,3,5-Trichlor-1,3,5-triazin-2,4,6-trion, 1,3,5-Tribrom-1,3,5-triazin-2,4,6-trion oder 1,3-Dibrom-1,3,5-triazin-2,4,6-trion. Besonders bevorzugt sind die halogenierenden Verbindungen ausgewählt aus den *N*-Halogensuccinimiden oder 1,3-Dihalogen-5,5-dimethylhydantoinen, ganz besonders bevorzugt sind 1,3-Dihalogen-5,5-dimethylhydantoine.

Weiterhin sind die halogenierenden Verbindungen besonders bevorzugt ausgewählt aus *N*-Bromsuccinimid (NBS), *N*-Iodsuccinimid (NIS), 1,3-Dibrom-5-5-dimethylhydantoin (DBDMH), 1,3-Diiod-5-5-dimethylhydantoin (DIDMH), 1,3,5-Tribrom-1,3,5-triazin-2,4,6-trion oder 1,3-Dibrom-1,3,5-triazin-2,4,6-trion, ganz besonders bevorzugt ist N-Iodsuccinimid (NIS), 1,3-Diiod-5-5-dimethylhydantoin (DIDMH) oder 1,3-Dibrom-5-5-dimethylhydantoin (DBDMH) und hervorgehoben ist *N*-Iodsuccinimid (NIS) und 1,3-Diiod-5,5-dimethylhydantoin (DIDMH).

Die halogenierenden Verbindungen können alleine oder in Kombination von zwei oder mehr eingesetzt werden, solange die eingesetzten Verbindungen dasselbe Halogen tragen.

Die halogenierende Verbindung kann erfindungsgemäß in einem Anteil zwischen 1,0 und 2,0 Äquivalenten (*Mono-Halogenverbindungen*) bzw. zwischen 0,5 und 1,0 Äquivalenten (Dihalogenverbindungen) und bevorzugt zwischen 1,1 und 1,2 Äquivalenten (Monohalogenverbindungen) bzw. zwischen 0,55 und 0,8 Äquivalenten (Dihalogenverbindungen), bezogen auf die gesamte eingesetzte Stoffmenge an Verbindung (II), eingesetzt werden.

Die halogenierende Verbindung kann erfindungsgemäß in Reinform als Feststoff oder als Suspension oder Lösung in einem geeigneten, unter den Reaktionsbedingungen inerten organischen Lösungsmittel, insbesondere in dem zuvor für die Reaktion ausgewählten Lösungsmittel vorliegen, bevorzugt in einer Konzentration von 40-90 Gew.%, besonders bevorzugt in einer Konzentration von 60-95 Gew.%. Geeignete organische Lösungsmittel sind insbesondere die für das Gesamtverfahren bevorzugten Lösungsmittel.

Geeignete Säuren sind erfindungsgemäß ausgewählt aus Mineralsäuren, Sulfonsäuren, Carbonsäuren und Lewis-Säuren.

Der Begriff Mineralsäuren umfasst erfindungsgemäß alle nicht kohlenstoffhaltigen, anorganischen Säuren, wie beispielsweise HF, HCl, HBr, HI, H₂SO₄, HNO₃, und H₃PO₄.

Geeignete Mineralsäuren sind bevorzugt ausgewählt aus HCl, HF, HNO₃, H₂SO₄ und H₃PO₄, besonders bevorzugt aus HNO₃, HF und H₂SO₄ und ganz besonders bevorzugt ist H₂SO₄.

Der Begriff Sulfonsäuren umfasst erfindungsgemäß die dem Fachmann allgemein bekannten gegebenenfalls substituierten Aryl- und Alkylsulfonsäuren, wie beispielsweise Methansulfonsäure, Trifluormethansulfonsäure, Benzolsulfonsäure und p-Toluolsulfonsäure.

Geeignete Sulfonsäuren sind bevorzugt ausgewählt aus Methansulfonsäure, Trifluormethansulfonsäure, Benzolsulfonsäure und para-Toluolsulfonsäure, besonders bevorzugt aus Methansulfonsäure und para-Toluolsulfonsäure und ganz besonders bevorzugt ist Methansulfonsäure.

Der Begriff Carbonsäuren umfasst erfindungsgemäß alle dem Fachmann allgemein bekannten Kohlenstoff-haltigen Säuren, die mindestens eine Carboxy-Gruppe (-COOH) enthalten, wie beispielsweise gegebenenfalls substituierte Alkyl- und Arylcarbonsäuren, sowie gegebenenfalls substituierte Alkyl- und Aryl-dicarbonsäuren.

Geeignete Carbonsäuren weisen bevorzugt einen pks-Wert von ≤ 5, besonders bevorzugt ≤ 3 auf.

Geeignete Carbonsäuren sind bevorzugt ausgewählt aus Essigsäure, Propionsäure, Trifluoressigsäure und Trichloressigsäure, besonders bevorzugt aus Essigsäure, Trifluoressigsäure und Trichloressigsäure und ganz besonders bevorzugt ist Essigsäure oder Trifluoressigsäure.

Der Begriff Lewis Säuren umfasst erfindungsgemäß die dem Fachmann allgemein bekannten anorganischen und organischen elektrophilen Elektronenpaarakzeptoren, insbesondere wasserfreie oder hydratisierte, anorganische Salze von

Lithium oder Erdalkalimetallen, insbesondere Mg und Ca, beispielsweise als Fluorid-, Chlorid- oder Bromid-Salze, Nitrate, Acetate, Sulfate oder Trifluormethansulfonate (OTf), bevorzugt als Nitrate oder Trifluormethansulfonate (OTf),

Metallen der Borgruppe, insbesondere Al, B oder In, beispielsweise als Fluorid-, Chlorid- oder Bromid-Salze, Nitrate, Acetate, Sulfate oder Trifluormethansulfonate (OTf), bevorzugt als Fluorid-, Chlorid- oder Bromid-Salze, Nitrate oder Trifluormethansulfonate (OTf),
und von Übergangsmetallen, insbesondere Fe, Zn, Cu, Sc, Ti oder Co, beispielsweise als Fluorid-, Chlorid- oder Bromid-Salze, Nitrate, Acetate, Sulfate oder Trifluormethansulfonate (OTf), bevorzugt als Nitrate oder Trifluormethansulfonate (OTf).

Die Salze können in wasserfreier Form, aber auch in ihrer hydratisierten Form, insbesondere mit gebundenem Kristallwasser erfindungsgemäß eingesetzt werden.

Die Verwendung anderer Metallsalze ist aus technischer Sicht zwar möglich, jedoch aus wirtschaftlicher sowie toxikologischer Sicht nicht bevorzugt.

Geeignete Lewis Säuren sind bevorzugt wasserfreie oder hydratisierte Salze ausgewählt aus den Fluorid-, Chlorid- oder Bromid-Salzen, Nitrate oder Trifluormethansulfonate (OTf) der Metalle B oder Al, aus den Nitraten oder Trifluormethansulfonaten (OTf) der Erdalkalimetalle Mg oder Ca oder den Nitraten oder Trifluormethansulfonaten (OTf) der Übergangsmetalle Fe, Zn, Cu oder Sc. Besonders bevorzugte Lewis Säuren sind wasserfreie oder hydratisierte Salze, ausgewählt aus Mg(NO₃)₂, Ca(NO₃)₂, Fe₂(NO₃)₃, Zn(NO₃)₂, Zn(OTf)₂, Cu(NO₃)₂, Sc(NO₃)₃, Ca(OTf)₂, Mg(OTf)₂, Cu(OTf)₂, BBr₃, BCl₃, BF₃^{∗}OEt₂, Al(NO₃)₃, Al(OTf)₃, Fe(OTf)₃, Cu(OTf)₂ und Sc(OTf)₃, ganz besonders bevorzugt aus Ca(OTf)₂, Mg(OTf)₂, Mg(NO₃)₂, Ca(NO₃)₂, Fe₂(NO₃)₃ und Fe(OTf)₃.

Geeignete Säuren sind erfindungsgemäß bevorzugt ausgewählt aus HF, HCl, HBr, HI, H₂SO₄, HNO₃, und H₃PO₄, gegebenenfalls substituierten Aryl- und Alkylsulfonsäuren, gegebenenfalls substituierte Alkyl- und Arylcarbonsäuren, gegebenenfalls substituierte Alkyl- und Aryl-dicarbonsäuren, wobei die Carbonsäuren einen pks-Wert von ≤ 5 aufweisen, und wasserfreien oder hydratisierten Fluorid-, Chlorid- oder Bromid-Salzen, Nitraten, Acetaten, Sulfaten oder Trifluormethansulfonaten (OTf) von Lithium oder der Erdalkalimetalle, insbesondere Mg und Ca, der Metalle der Borgruppe, insbesondere Al, B oder In, und der Übergangsmetalle, insbesondere Fe, Zn, Cu, Sc, Ti oder Co.

Geeignete Säuren sind erfindungsgemäß besonders bevorzugt ausgewählt aus HCl, HF, HNO₃, H₂SO₄, H₃PO₄, Methansulfonsäure, Trifluormethansulfonsäure, Benzolsulfonsäure, para-Toluolsulfonsäure, Essigsäure, Propionsäure, Trifluoressigsäure,Trichloressigsäure und den wasserfreien oder hydratisierten Salzen ausgewählt aus den Fluorid-, Chlorid- oder Bromid-Salzen, Nitrate oder Trifluormethansulfonate (OTf) der Metalle B oder Al, aus den Nitraten oder Trifluormethansulfonaten (OTf) der Erdalkalimetalle Mg oder Ca oder den Nitraten oder Trifluormethansulfonaten (OTf) der Übergangsmetalle Fe, Zn, Cu oder Sc.

Geeignete Säuren sind erfindungsgemäß ganz besonders bevorzugt ausgewählt aus HNO₃, HF, H₂SO₄, Methansulfonsäure, para-Toluolsulfonsäure, Essigsäure, Trifluoressigsäure, Trichloressigsäure, Mg(NO₃)₂, Ca(NO₃)₂, Fe₂(NO₃)₃, Zn(NO₃)₂, Zn(OTf)₂, Cu(NO₃)₂, Sc(NO₃)₃, Ca(OTf)₂, Mg(OTf)₂, Cu(OTf)₂, BBr₃, BCl₃, BF₃^{∗}OEt₂, Al(NO₃)₃, Al(OTf)₃, Fe(OTf)₃, Cu(OTf)₂ und Sc(OTf)₃.

Geeignete Säuren sind erfindungsgemäß hervorgehoben ausgewählt aus H₂SO₄, Methansulfonsäure, Essigsäure, Trifluoressigsäure, Mg(NO₃)₂, Ca(NO₃)₂, Ca(OTf)₂, Mg(OTf)₂, Fe₂(NO₃)₃ und Fe(OTf)₃.

Weitere bevorzugte Ausgestaltungen des erfindungsgemäßen Verfahrens betreffend die verwendeten Säure sind im Folgenden aufgeführt.

Der Begriff Mineralsäuren umfasst erfindungsgemäß alle nicht kohlenstoffhaltigen, anorganischen Säuren, wie beispielsweise HF, HCl, HBr, HI, H₂SO₄, HNO₃, und H₃PO₄.

Geeignete Mineralsäuren sind bevorzugt ausgewählt aus HCl, H₂SO₄ und H₃PO₄, besonders bevorzugt aus H₂SO₄ und H₃PO₄ und ganz besonders bevorzugt ist H₂SO₄.

Der Begriff Sulfonsäuren umfasst erfindungsgemäß die dem Fachmann allgemein bekannten gegebenenfalls substituierten Aryl- und Alkylsulfonsäuren, wie beispielsweise Methansulfonsäure, Trifluormethansulfonsäure, Benzolsulfonsäure und p-Toluolsulfonsäure.

Geeignete Sulfonsäuren sind bevorzugt ausgewählt aus Methansulfonsäure, Trifluormethansulfonsäure und para-Toluolsulfonsäure, besonders bevorzugt aus Methansulfonsäure und Trifluormethansulfonsäure und ganz besonders bevorzugt ist Methansulfonsäure.

Der Begriff Carbonsäuren umfasst erfindungsgemäß alle dem Fachmann allgemein bekannten Kohlenstoff-haltigen Säuren, die mindestens eine Carboxy-Gruppe (-COOH) enthalten, wie beispielsweise gegebenenfalls substituierte Alkyl- und Arylcarbonsäuren, sowie gegebenenfalls substituierte Alkyl- und Aryl-dicarbonsäuren.

Geeignete Carbonsäuren weisen bevorzugt einen pks-Wert von ≤ 5, besonders bevorzugt ≤ 3 auf.

Geeignete Carbonsäuren sind bevorzugt ausgewählt aus Essigsäure, Propionsäure, Trifluoressigsäure und Trichloressigsäure, besonders bevorzugt aus Trifluoressigsäure und Trichloressigsäure.

Der Begriff Lewis Säuren umfasst erfindungsgemäß die dem Fachmann allgemein bekannten anorganischen und organischen elektrophilen Elektronenpaarakzeptoren, wie beispielsweise wasserfreie, anorganische Salze von Lithium, Erdalkalimetallen (insbesondere Mg und Ca), Metallen der Borgruppe (insbesondere Al, B oder In) und von Übergangsmetallen (insbesondere Fe, Zn, Cu, Sc, Ti oder Co), beispielsweise als Fluorid-, Chlorid- oder Bromid-Salze oder Trifluormethansulfonate (OTf). Die Verwendung anderer Metallsalze ist aus technischer Sicht zwar möglich, jedoch aus wirtschaftlicher sowie toxikologischer Sicht nicht bevorzugt.

Geeignete Lewis Säuren sind bevorzugt ausgewählt aus Verbindungen der Borgruppe, insbesondere aus BBr₃, BCl₃ und BF₃^{∗}OEt₂, aus Salzen der Erdalkalimetalle, insbesondere Mg(OTf)₂ und Ca(OTf)₂, sowie der Übergangsmetallsalzen, insbesondere Zn(OTf)₂, Fe(OTf)₃, Cu(OTf)₂, Sc(OTf)₃, besonders bevorzugt sind Fe(OTf)₃ und Sc(OTF)₃, ganz besonders bevorzugt ist Fe(OTf)₃.

In einer besonders bevorzugten Ausgestaltung der vorliegenden Erfindung sind die geeigneten Säuren ausgewählt aus HCl, H₂SO₄, H₃PO₄, Methansulfonsäure, Trifluormethansulfonsäure, para-Toluolsulfonsäure, Essigsäure, Propionsäure, Trifluoressigsäure, Trichloressigsäure, Mg(OTf)₂, Zn(OTf)₂, Fe(OTf)₃, Cu(OTf)₂, Sc(OTf)₂, BBr₃, BCl₃ und BF₃^{∗}OEt₂, ganz besonders bevorzugt aus H₂SO₄, H₃PO₄, Methansulfonsäure, Trifluormethansulfonsäure, Trifluoressigsäure, Trichloressigsäure, Essigsäure, Sc(OTf)₂ oder Fe(OTf)₃, insbesondere bevorzugt aus H₂SO₄, Methansulfonsäure, Trifluoressigsäure oder Fe(OTf)₃.

Die Säuren können alleine oder in Kombination von zwei oder mehreren Säuren eingesetzt werden.

Die Säure kann erfindungsgemäß bevorzugt als Reinstoff oder als Lösung in einem geeigneten, unter den Reaktionsbedingungen inerten, organischen Lösungsmittel, insbesondere dem zuvor für die Reaktion ausgewählten Lösungsmittel, bevorzugt in einer Konzentration von > 30 Gew.%, besonders bevorzugt in einer Konzentration von > 60 Gew.%, eingesetzt werden. Geeignete organische Lösungsmittel sind insbesondere die für das Gesamtverfahren bevorzugten Lösungsmittel.

Besonders bevorzugt wird die Säure jedoch als Reinstoff, sowie bei Mineralsäuren in deren kommerziell erhältlicher, konzentrierter Form ohne weitere Verdünnung eingesetzt.

Bevorzugt wird die Säure nicht (zusätzlich zur kommerziell erhältlichen Form) verdünnt mit Wasser eingesetzt.

Die Säure wird erfindungsgemäß in einem Anteil von ≥ 0,0001 Äquivalenten und < 0,3 Äquivalenten bevorzugt ≥ 0,001 Äquivalenten und ≤ 0,15 Äquivalenten und ganz besonders bevorzugt ≥ 0,005 Äquivalenten und ≤ 0,05 Äquivalenten, bezogen auf die gesamte eingesetzte Stoffmenge an Verbindung (II), eingesetzt.

Die Reaktion wird bevorzugt in einem Temperaturbereich von -78 bis 200 °C, besonders bevorzugt bei Temperaturen zwischen -20 bis 100 °C und ganz besonders bevorzugt zwischen 0 °C und 50 °C durchgeführt.

Die Reaktion kann unter erhöhtem als auch vermindertem Druck ausgeführt werden. Bevorzugt wird sie aber unter Normaldruck durchgeführt, z.B. im Bereich von 1013 hPa±300 hPa, oder im Bereich von 1013 hPa±100 hPa, oder im Bereich von 1013 hPa±50 hPa.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Nitrile wie Acetonitril, Propionitril, Butyronitril, Isobutyronitril, Benzonitril, m-Chlorbenzonitril, aliphatische, cycloaliphatische oder aromatische Kohlenwasserstoffe (z.B. Pentan, Hexan, Heptan, Oktan, Nonan und technische Kohlenwasserstoffe, Cyclohexan, Methylcyclohexan, Petrolether, Ligroin, Benzol, Toluol, Anisol, Xylol, Mesitylen, Nitrobenzol), Ester (z.B. Methyl-, Ethyl-, Isopropyl-, Butyl-, Isobutylacetat, Dimethyl-, Dibutyl-, Ethylencarbonat); Amide (z.B. N,N-Dimethylformamid (DMF), N,N-Dipropyl-formamid, N,N-Dibutyl-formamid (DBF), N,N-Dimethylacetamid (DMAC), N-Methyl-pyrrolidon (NMP), aliphatische oder cycloaliphatische Ether (z.B. 1,2-Dimethoxyethan (DME), Diglyme, Tetrahydrofuran (THF), 2-Methyl-THF, 1,4-Dioxan, Methyl-tert-Butylether), Carbonsäuren (z.B. Essigsäure, *n*-Propansäure, *n*-Butansäure), Ketone (z.B. Aceton, Ethylmethylketon, Methyl-Isobutylketon).

Bevorzugte Verdünnungs- bzw. Lösungsmittel sind aromatische Kohlenwasserstoffe, insbesondere Benzol, Toluol, Xylol, Mesitylen, Chlorbenzol oder Dichlorbenzol; halogenierte Kohlenwasserstoffe, insbesondere Dichlormethan, Chloroform, 1,2-Dichlorethan oder Tetrachlorkohlenstoff; Ester, insbesondere Ethylacetat, Isopropylacetat und Butylacetat; Amide, insbesondere DMF, DMAC und NMP; Nitrile, insbesondere Acetonitril oder Propionitril; oder Carbonsäuren, insbesondere Essigsäure oder n-Propansäure.

In einer besonders bevorzugten Ausführungsform ist das Lösungsmittel ein Nitril, insbesondere Acetonitril, oder eine Carbonsäure, insbesondere Essigsäure.

Die Lösungsmittel können allein oder in Kombination von zwei oder mehr eingesetzt werden.

Die Dauer der Halogenierung der Verbindungen der Formel (II) ist kurz und liegt bevorzugt im Bereich von 0,15 h bis 5 h, besonders bevorzugt im Bereich von 0,25 bis 3h. Eine längere Reaktionsdauer ist möglich, jedoch aus wirtschaftlicher Sicht nicht sinnvoll.

Die halogenierende Verbindung kann erfindungsgemäß in Reinform als Feststoff oder als Suspension oder Lösung zu einer Lösung der Verbindung der allgemeinen Formel (II) zugegeben werden.

In einer bevorzugten Ausgestaltung des erfindungsgemäßen Verfahrens wird jedoch eine Lösung der Verbindung der allgemeinen Formel (II) zu einer Suspension oder Lösung der halogenierenden Verbindung in einem erfindungsgemäßen Lösungs- oder Verdünnungsmittel zu dosiert.

Es kommen dabei bevorzugt die oben genannten erfindungsgemäß bevorzugten Lösungs- oder Verdünnungsmittel zum Einsatz.

Die Dauer der Dosierung kann in einem bevorzugten Bereich von 0,5 bis 6 Stunden, besonders bevorzugt von 1 bis 4 Stunden liegen. Längere Dosierzeiten sind aus technischer Sicht auch möglich, sind jedoch aus wirtschaftlicher Sicht nicht sinnvoll.

Die Dosierung erfolgt bevorzugt in einem Temperaturbereich von -78 bis 200 °C, besonders bevorzugt bei Temperaturen zwischen -20 bis 100 °C und ganz besonders bevorzugt zwischen 0 °C und 50 °C. In einer vorteilhaften Ausgestaltung entspricht die Temperatur, bei der dosiert wird, der Reaktionstemperatur.

In einer bevorzugten Ausgestaltung des erfindungsgemäßen Verfahrens werden die Verbindungen der allgemeinen Formel (II) in einem geeigneten organischen Lösungsmittel mit 1,3-Diiod-5,5-dimethylhydantoin (DIDMH) unter Zusatz von 0,2 Äquivalenten einer der oben genannten erfindungsgemäß bevorzugten Säuren, bezogen auf die gesamte eingesetzte Stoffmenge der Verbindung (II), bei 1013 hPa±300 hPa und einer Temperatur von -20 bis 100°C zu Verbindungen der allgemeinen Formel (I) umgesetzt. Die Reaktionsmischung wird für eine Dauer von 0,15 bis 6 Stunden unter denselben Bedingungen gerührt. Die Reaktionsdauer wird dabei bevorzugt so gewählt, dass die Reaktion bei mittels HPLC^{a)} ermittelten vollständigen Umsatz beendet wird.

In einer besonders bevorzugten Ausgestaltung des erfindungsgemäßen Verfahrens werden die Verbindungen der allgemeinen Formel (II) zusammen mit 0,05 Äquivalenten Schwefelsäure, bezogen auf die gesamte eingesetzte Stoffmenge der Verbindung (II), als Lösung in Acetonitril zu einer Suspension von 1,3-Diiod-5,5-dimethylhydantoin (DIDMH) in Acetonitril bei 1013 hPa±50 hPa und einer Temperatur von 0 bis 50°C zwischen 0,25 und 3 h zu dosiert. Die Reaktionsmischung wird für eine Dauer von 0,25 bis 6 Stunden unter denselben Bedingungen gerührt. Die Reaktionsdauer wird dabei bevorzugt so gewählt, dass die Reaktion bei mittels HPLC^{a)} ermittelten vollständigen Umsatz beendet wird.

Bevorzugt werden die Verbindungen der Formel (I) im erfindungsgemäßen Verfahren im Anschluss an die Umsetzung isoliert und aufgearbeitet.

Zur Isolierung und Aufarbeitung der Halogenpyrazole der allgemeinen Formel (I) kann überschüssiges Halogenierungsmittel durch Zugabe von dem Fachmann bekannten, geeigneten Reduktionsmitteln (z.B. Natriumsulfit oder Natriumthiosulfat) unschädlich gemacht werden. Das Reduktionsmittel kann dabei in Reinform als Feststoff oder als gesättigte, wässrige Lösung zugegeben werden. Das Produkt kann direkt oder nach teilweiser Entfernung des Lösungsmittel, beispielsweise nach Entfernung von 50% des Lösungsmittels, durch Verwässerung des Reaktionsgemisches ausgefällt und durch Filtration isoliert werden. Alternativ kann das Produkt in ein organisches Lösungsmittel extrahiert und nach wässriger Aufarbeitung und anschließender Entfernung des Lösungs- oder Extraktionsmittels isoliert werden.

### Beispiele

Die nachfolgenden Beispiele erläutern das erfindungsgemäße Verfahren näher, ohne die Erfindung dabei auf diese einzuschränken.

### 1) 1-[2,6-Dichlor-4-(1,1,1,2,3,3,3-heptafluorpropan-2-yl)phenyl]-4-iod-1H-pyrazol (I-1)

11,1 g (28,0 mmol, 0,5 eq) 1,3 Diiodo-5,5-dimethylhydantoin wurden in 25mL Acetonitril vorgelegt und bei 20 °C Innentemperatur über 0,5 h mit einer Lösung von 22,7 g (Reinheit: 93%, 55,6 mmol, 1,0 eq) 1[2,6-Dichloro-4-[1,2,2,2-tetrafluoro-1-(trifluoromethyl)ethyl]phenyl]pyrazol und 0,28g (2,8mmol, 0,05 eq) 96% Schwefelsäure, gelöst in 25 mL Acetonitril, versetzt. Nach vollständiger Zugabe wurde 10 min weiter gerührt und im Anschluss ein vollständiger Umsatz zum Iodpyrazol mittels HPLC^{a)} detektiert. Dann wurden 10 mL Wasser zugegeben und die Reaktion durch Zugabe von 5 mL gesättigter Natriumsulfit-Lösung abgebrochen. Das Lösungsmittel wurde teilweise im Vakuum abdestilliert und das Produkt nach Fällung mit 20 mL Wasser filtriert. Der Rückstand wurde zweimal mit je 80 mL Wasser gewaschen und nach Trocknung im Vakuum bei 40 °C wurde das Produkt als farbloser bis gelblicher Feststoff erhalten: Ausbeute 29,1g (98% d. Theorie).

¹H-NMR (CDCl₃, 400 MHz) δ (ppm) = 7,84 ppm (s, 1H); 7,71 ppm (s, 2H); 7,65 ppm (s, 1H).

### 2) 1-[2,6-Dichlor-4-(1,1,1,2,3,3,3-heptafluorpropan-2-yl)phenyl]-4-iod-1H-pyrazol (I-1)

5,1 g (12,9 mmol, 0,505 eq) 1,3 Diiodo-5,5-dimethylhydantoin wurden in 10 mL Acetonitril vorgelegt und bei 20 °C Innentemperatur über 15 min mit einer Lösung von 10,0 g (Reinheit: 98%, 25,7 mmol, 1,0 eq) 1[2,6-Dichloro-4-[1,2,2,2-tetrafluoro-1-(trifluoromethyl)ethyl]phenyl]pyrazol und 80 mg (1,3 mmol, 0,05 eq) **Eisessig,** gelöst in 10 mL Acetonitril, versetzt. Nach vollständiger Zugabe wurde die Reaktion auf 50 °C erhitzt und bei dieser Temperatur weiter gerührt. Nach 10 h konnte mittels HPLC^{a)} ein Umsatz von 90% zum Iodpyrazol detektiert werden. Die Reaktion wrude durch Zugabe von 5 mL gesättigter Natriumsulfit-Lösung abgebrochen und das Produkt nach Fällung mit 100 mL Wasser filtriert. Der Rückstand wurde zweimal mit je 20 mL Wasser gewaschen und nach Trocknung im Vakuum bei 40 °C wurde das Produkt als schwach oranger Feststoff erhalten: Ausbeute 12,1g (82% d. Theorie).

### 3) 1-[2,6-Dichlor-4-(1,1,1,2,3,3,3-heptafluorpropan-2-yl)phenyl]-4-iod-1H-pyrazol (I-1)

51,4 g (129,9 mmol, 0,505 eq) 1,3 Diiodo-5,5-dimethylhydantoin wurden in 100 mL Acetonitril vorgelegt und bei 20 °C Innentemperatur über 0,5 h mit einer Lösung von 100,0 g (Reinheit: 98%, 257,2 mmol, 1,0 eq) 1-[2,6-Dichloro-4-[1,2,2,2-tetrafluoro-1-(trifluoromethyl)ethyl]phenyl]pyrazol und 26 mg (0,26 mmol, **0,001 eq)** 96% Schwefelsäure, gelöst in 100 mL Acetonitril, versetzt. Nach vollständiger Zugabe wurde die Reaktionsmischung bei 50 °C gerührt. Nach 1 h konnte ein vollständiger Umsatz zum Iodpyrazol mittels HPLC^{a)} detektiert werden. Dann wurden 50 mL Wasser zugegeben und die Reaktion durch Zugabe von 50 mL gesättigter Natriumsulfit-Lösung abgebrochen. Das Lösungsmittel wurde teilweise im Vakuum abdestilliert und das Produkt nach Fällung mit 300 mL Wasser filtriert. Der Rückstand wurde zweimal mit je 100 mL Wasser gewaschen und nach Trocknung im Vakuum bei 40 °C wurde das Produkt als farbloser bis gelblicher Feststoff erhalten: Ausbeute 129,5g (94% d. Theorie).

### 4) 1-[2,6-Dichlor-4-(1,1,1,2,3,3,3-heptafluorpropan-2-yl)phenyl]-4-iod-1H-pyrazol (I-1)

5,1 g (12,9 mmol, 0,505 eq) 1,3 Diiodo-5,5-dimethylhydantoin wurden in 10 mL Acetonitril vorgelegt und bei 20 °C Innentemperatur über 15 min mit einer Lösung von 10,0 g (Reinheit: 98%, 25,7 mmol, 1,0 eq) 1[2,6-Dichloro-4-[1,2,2,2-tetrafluoro-1-(trifluoromethyl)ethyl]phenyl]pyrazol und 134 mg (0,26 mmol, **0,01 eq) Fe(OTf)₃,** gelöst in 10 mL Acetonitril, versetzt. Nach vollständiger Zugabe wurde die Reaktionsmischung weiter bei RT gerührt. Nach 2 h konnte ein vollständiger Umsatz zum Iodpyrazol mittels HPLC^{a)} detektiert werden. Dann wurde die Reaktion durch Zugabe von 5 mL gesättigter Natriumsulfit-Lösung abgebrochen und das Produkt nach Fällung mit 100 mL Wasser filtriert. Der Rückstand wurde zweimal mit je 100 mL Wasser gewaschen und nach Trocknung im Vakuum bei 40 °C wurde das Produkt als farbloser bis gelblicher Feststoff erhalten: Ausbeute 12,7g (88% d. Theorie).

### 5) 1-[2,6-Dichlor-4-(1,1,1,2,3,3,3-heptafluorpropan-2-yl)phenyl]-4-iod-1H-pyrazol (I-1)

6,3 g (27,0 mmol, 1,05 eq) **N-Iodsuccinimid** wurden in 10mL Acetonitril vorgelegt und bei 20 °C Innentemperatur über 15 min mit einer Lösung von 10,0 g (Reinheit: 98%, 25,7 mmol, 1,0 eq) 1-[2,6-Dichloro-4-[1,2,2,2-tetrafluoro-1-(trifluoromethyl)ethyl]phenyl]pyrazol und 131 mg (1,29 mmol, 0,05 eq) 96% Schwefelsäure, gelöst in 10 mL Acetonitril, versetzt. Nach vollständiger Zugabe wurde die Reaktionsmischung bei dieser Temperatur gerührt. Nach 1 h konnte ein vollständiger Umsatz zum Iodpyrazol mittels HPLC^{a)} detektiert werden. Dann wurde die Reaktion durch Zugabe von 5 mL gesättigter Natriumsulfit-Lösung abgebrochen und das Produkt nach Fällung mit 100 mL Wasser filtriert. Der Rückstand wurde zweimal mit je 100 mL Wasser gewaschen und nach Trocknung im Vakuum bei 40 °C wurde das Produkt als farbloser bis gelblicher Feststoff erhalten: Ausbeute 12,7g (94% d. Theorie).

### 6) 1-[2,6-Dichlor-4-(1,1,1,2,3,3,3-heptafluorpropan-2-yl)phenyl]-4-iod-1H-pyrazol (I-1)

10,0 g (Reinheit: 97,4%, 25,5 mmol, 1,0 eq) 1-[2,6-Dichloro-4-[1,2,2,2-tetrafluoro-1-(trifluoromethyl)ethyl]phenyl]pyrazol wurden in 20 mL Acetonitril gelöst und mit 0,26 mg (2,55 µmol, 0,0001 eq) 96% H₂SO₄ versetzt. Nach Zugabe von 1,53 g (3,9 mmol, 0,15 eq) 1,3 Diiodo-5,5-dimethylhydantoin wurde die Lösung auf 60 °C erhitzt und bei dieser Temperatur gerührt. Nach 5 h wurde erneut 1,53 g (3,9 mmol, 0,15 eq) 1,3 Diiodo-5,5-dimethylhydantoin zugegeben und weiter bei 60 °C gerührt und die Zugabe nach insgesamt 10 h mit weiteren 2,04 g (5,2 mmol, 0,20 eq) 1,3 Diiodo-5,5-dimethylhydantoin wiederholt. Nach insgesamt 17 h bei 60 °C konnte ein Umsatz von 99% zum Iodpyrazol mittels HPLC^{a)} detektiert werden. Das Produkt wurde nicht isoliert.

### 7) 4-Brom-1-[2,6-dichlor-4-(1,1,1,2,3,3,3-heptafluorpropan-2-yl)phenyl]-1H-pyrazol (I-2)

3,86 g (13,2 mmol, 0,51 eq) 1,3 Dibromo-5,5-dimethylhydantoin wurden in 50mL Acetonitril vorgelegt und bei 20 °C Innentemperatur über 0,5 h mit einer Lösung von 10,0 g (Reinheit: 99%, 26,2 mmol, 1,0 eq) 1-[2,6-Dichloro-4-[1,2,2,2-tetrafluoro-1-(trifluoromethyl)ethyl]phenyl]pyrazol und 0,13 g (1,3 mmol, 0,05 eq) 96% Schwefelsäure, gelöst in 50 mL Acetonitrile versetzt. Nach vollständiger Zugabe wurde 10 min weiter gerührt und im Anschluss ein vollständiger Umsatz zum Brompyrazol mittels HPLC^{a)} detektiert. Dann wurden 10 mL Wasser zugegeben und die Reaktion durch Zugabe von 5mL gesättigter Natriumsulfit-Lösung abgebrochen. Das Lösungsmittel wurde teilweise im Vakuum abdestilliert und das Produkt nach Fällung mit 20 mL Wasser filtriert. Der Rückstand wurde zweimal mit je 80 mL Wasser gewaschen und nach Trocknung im Vakuum bei 40 °C wurde das Produkt als farbloser Feststoff erhalten: Ausbeute 11,6 g (96% d. Theorie).

¹H-NMR (CDCl₃, 400 MHz): δ (ppm) = 7,80 ppm (s, 1H); 7,71 ppm (s, 2H); 7,63 ppm (s, 1H)

### 8) 4-Brom-1-[2,6-dichlor-4-(1,1,1,2,3,3,3-heptafluorpropan-2-yl)phenyl]-1H-pyrazol (I-2)

4,81 g (27,2 mmol, 1,05 eq) **N-Bromsuccinimid** wurden in 10 mL Acetonitril vorgelegt und bei 20 °C Innentemperatur über 15 min mit einer Lösung von 10,0 g (Reinheit: 99%, 26,2 mmol, 1,0 eq) 1-[2,6-Dichloro-4-[1,2,2,2-tetrafluoro-1-(trifluoromethyl)ethyl]phenyl]pyrazol und 0,13 g (1,3 mmol, 0,05 eq) 96% Schwefelsäure, gelöst in 10 mL Acetonitril versetzt. Nach vollständiger Zugabe wurde bei dieser Temperatur weiter gerührt und nach 1 h ein vollständiger Umsatz zum Brompyrazol mittels HPLC^{a)} detektiert. Dann wurde die Reaktion durch Zugabe von 5mL gesättigter Natriumsulfit-Lösung abgebrochen und das Produkt nach Fällung mit 100 mL Wasser filtriert. Der Rückstand wurde zweimal mit je 20 mL Wasser gewaschen und nach Trocknung im Vakuum bei 40 °C wurde das Produkt als farbloser Feststoff erhalten: Ausbeute 11,8 g (96% d. Theorie).

### 9) 4-Brom-1-[2,6-dichlor-4-(1,1,1,2,3,3,3-heptafluorpropan-2-yl)phenyl]-1H-pyrazol (I-2)

2,0 g (7,1 mmol, 0,55 eq) **Dibromisocyanursäure** wurden in 10 mL Acetonitril vorgelegt und bei 20 °C Innentemperatur mit 5,0 g (Reinheit: 98%, 12,9 mmol, 1,0 eq) 1-[2,6-Dichloro-4-[1,2,2,2-tetrafluoro-1-(trifluoromethyl)ethyl]phenyl]pyrazol und 63 mg (0,6 mmol, 0,05 eq) 96% Schwefelsäure versetzt. Nach vollständiger Zugabe wurde bei dieser Temperatur weiter gerührt und nach 30 min ein vollständiger Umsatz zum Brompyrazol mittels HPLC^{a)} detektiert. Dann wurde die Reaktion durch Zugabe von 5mL gesättigter Natriumsulfit-Lösung abgebrochen und die entstandende Isocyanursäure nach Verdünnung mit 20 mL Acetonitril mittels Filtration entfernt. Die Mutterlauge wurde tropfenweise mit 150 mL Wasser versetzt und der ausgefallene Feststoff filtriert. Der Rückstand wurde zweimal mit je 30 mL Wasser gewaschen und nach Trocknung im Vakuum bei 40 °C wurde das Produkt als farbloser Feststoff erhalten: Ausbeute 5,8 g (95% d. Theorie).

### Weitere Versuche zu den Säuren:

Tabelle 1) gibt eine Übersicht über weitere Versuche die analog zu Versuch 1) durchgeführt wurden und die erzielten Umsätze der Verbindung (I-1) bestimmt mittels HPLC^{a)}.

Dabei wurden die Art der verwendeten Säure, die Temperatur und die Reaktionsdauer variiert. Alle übrigen Parameter und Reaktanden wurden beibehalten.

**Tabelle 1:**

| Säure | Temperatur (°C) | Zeit (h) | Umsatz (%) |
|---|---|---|---|
| HCl (37%) | 40 | 24 | 85 |
| H₃PO₄ | 40 | 11 | 89 |
| Trifluoressigsäure | 40 | 9 | 91 |
| para- Toluolsulfonsäure | 25 | 1 | 88 |
| Mg(OTf)₂ | 40 | 6,5 | 91 |
| Ca(OTf)₂ | 40 | 6,5 | 91 |
| Methansulfonsäure | 25 | 2 | 90 |
| Fe₂(NO₃)₃^{∗}9H₂O | 40 | 2 | 91 |
| Mg(NO₃)₂^{∗}6H₂O | 40 | 7 | 88 |
| BF₃^{∗}EtO₂ | 25 | 3 | 92 |
| MgSO₄^{∗}4H₂O | 40 | 12 | 60 |

### Vergleichsbeispiel ohne Säurezugabe:

### 1-[2,6-Dichlor-4-(1,1,1,2,3,3,3-heptafluorpropan-2-yl)phenyl]-4-iod-1H-pyrazol (I-1)

0,5 g (1,3 mmol, 1,0 eq) 1-[2,6-Dichloro-4-[1,2,2,2-tetrafluoro-1-(trifluoromethyl)ethyl]phenyl]pyrazol wurden in 10mL Acetonitril vorgelegt und mit 2,85 g (0.7mmol, 0,55 eq) 1,3 Diiodo-5,5-dimethylhydantoin versetzt. Die Reaktionsmischung wurde auf 65-70 °C erhitzt und bei dieser Temperatur 15 h gerührt. Nach dieser Zeit konnte mittels HPLC^{a)} ein Umsatz von 54% zum gewünschten iodierten Produkt festgestellt werden. Das Produkt wurde nicht isoliert.

Analog zu den Versuchen 1) und 7) konnten die folgenden halogenierten N-Arylpyrazole der allgemeinen Formel (I) hergestellt werden:

### 4-Brom-1-[2-brom-4-(1,1,1,2,3,3,3-heptafluorpropan-2-yl)-6-(trifluormethoxy)phenyl]-1H-pyrazol (I-3)

**Umsatz nach HPLC^{a)}: >99% (r.t., 1 h)**

¹H-NMR (CDCl₃, 400 MHz) δ (ppm) = 7.92 (d, J = 1.9 Hz, 1H), 7.79 (s, 1H), 7.63 (s, 2H).

### 4-Brom-1-[2-chlor-4-(1,1,1,2,3,3,3-heptafluorpropan-2-yl)-6-(trifluormethoxy)phenyl]-1H-pyrazol (I-4)

**Umsatz nach HPLC^{a)}: >99% (r.t., 1 h)**

¹H-NMR (CDCl₃, 400 MHz) δ (ppm) = 7.80 (d, J = 1.8 Hz, 1H), 7.79 (s, 1H), 7.64 (s, 1H), 7.59 (s, 1H).

### 4-Brom-1-[2-chloro-4-[1,2,2,2-tetrafluoro-1-(trifluoromethyl)ethyl]-6-(trifluoromethyl)phenyl]-1H-pyrazol (I-5)

**Umsatz nach HPLC^{a)}: >99% (r.t., 1 h)**

¹H-NMR (DMSO-d₆, 400 MHz) δ (ppm) = 8.48 (br s, 1H), 8.47 (s, 1H), 8.06 (br s, 1H), 8.03 (s, 1H).

### 4-Brom-1-[2-brom-4-[1,2,2,2-tetrafluoro-1-(trifluoromethyl)ethyl]-6-(trifluoromethyl)phenyl]-1H-pyrazol (I-6)

**Umsatz nach HPLC^{a)}: >99% (r.t., 4 h)**

¹H-NMR (CDCl₃, 400 MHz) δ (ppm) = 8.17 (br s, 1H), 7.99 (br s, 1H), 7.79 (s, 1H), 7.62 (s, 1H).

### 4-Brom-1-[2-methyl-4-[1,2,2,2-tetrafluoro-1-(trifluoromethyl)ethyl]-6-(trifluoromethyl)phenyl]-1H-pyrazol (I-7)

**Umsatz nach HPLC^{a)}: >99% (40 °C, 1 h)**

¹H-NMR (CDCl₃, 400 MHz) δ (ppm) = 7.87 (br s, 1H), 7.78 (br s, 1H), 7.7 ( s, 1H), 7.59 (s, 1H), 2.13 (s, 3H).

### 1-[2-Brom-4-(1,1,1,2,3,3,3-heptafluorpropan-2-yl)-6-(trifluormethoxy)phenyl]-4-iod-1H-pyrazol (I-8)

**Umsatz nach HPLC^{a)}: >99% (r.t., 0.5 h)**

¹H-NMR (CDCl₃, 400 MHz) δ (ppm) = 7.92 (d, J = 1.8 Hz, 1H), 7.83 (s, 1H), 7.65 (s, 1H), 7.63 (s, 1H).

### 1-[2-Chlor-4-(1,1,1,2,3,3,3-heptafluorpropan-2-yl)-6-(trifluormethoxy)phenyl]-4-iod-1H-pyrazol (I-9)

**Umsatz nach HPLC^{a)}: >99% (r.t., 0.5 h)**

¹H-NMR (CDCl₃, 400 MHz) δ (ppm) = 7.83 (d, J = 1.9 Hz, 1H), 7.77 (s, 1H), 7.66 (s, 1H), 7.59 (s, 1H).

### 1-[2-Chlor-4-[1,2,2,2-tetrafluoro-1-(trifluoromethyl)ethyl]-6-(trifluoromethyl)phenyl]-4-iod-1H-pyrazol (I-10)

**Umsatz nach HPLC^{a)}: >99% (r.t., 0.5 h)**

¹H-NMR (DMSO-d₆, 400 MHz) δ (ppm) = 8.47 (br s, 1H), 8.38 (s, 1H), 8.05 (br s, 1H), 7.97 (s, 1H).

### 1-[2-Bromo-4-[1,2,2,2-tetrafluoro-1-(trifluoromethyl)ethyl]-6-(trifluoromethyl)phenyl]-4-iod-1H-pyrazol (I-11)

**Umsatz nach HPLC^{a)}: >99% (r.t., 0.5 h)**

¹H-NMR (CDCl₃, 400 MHz) δ (ppm) = 8.16 (br s, 1H), 7.99 (br s, 1H), 7.83 (s, 1H), 7.64 (s, 1H).

### 4-Iod-1-[2-methyl-4-[1,2,2,2-tetrafluoro-1-(trifluoromethyl)ethyl]-6-(trifluoromethyl)phenyl]-1H-pyrazol (I-12)

**Umsatz nach HPLC^{a)}: >99% (r.t., 0.5 h)**

¹H-NMR (CDCl₃, 400 MHz) δ (ppm) = 7.87 (br s, 1H), 7.81 (s, 1H), 7.78 (br s, 1H), 7.61 (s, 1H), 2.11 (s, 3H).

### Methoden:

Die NMR-Daten der Beispiele werden in klassischer Form (δ-Werte, Multiplettaufspaltung, Anzahl der H-Atome) aufgeführt.

Das Lösungsmittel und die Frequenz, in welchem das NMR-Spektrum aufgenommen wurde, sind jeweils angegeben.

^{a)} HPLC (High Performance Liquid Chromatography) an einer Phasenumkehrsäule (C18).Agilent 1100 LC-System; Phenomex Prodigy 100 x 4mm ODS3; Eluent A: Acetonitril (0.25mL/L); Eluent B: Wasser (0.25mL TFA/L); linearer Gradient von 5 % Acetonitril bis 95 % Acetonitril in 7,00 min, dann 95% Acetonitril für weitere 1,00 min; Ofentemperatur 40 °C; Fluß:2,0 mL/min.

## Patentansprüche

1. Verfahren zur Herstellung von Verbindungen der Formel (I) 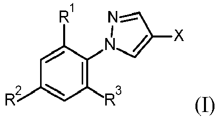 worin
X für Halogen steht;
R¹ für Wasserstoff, Cyano, Halogen, gegebenenfalls mit Halogen oder CN substituiertes C₁-C₄-Alkyl oder für gegebenenfalls mit Halogen substituiertes C₁-C₄-Alkoxy steht,
R² für Trifluormethylsulfonyl, Trifluormethylsulfinyl, Trifluormethylsulfanyl, Halogen, gegebenenfalls mit Halogen substituiertes C₁-C₄-Alkyl oder für gegebenenfalls mit Halogen substituiertes C₁-C₄-Alkoxy steht und
R³ für Wasserstoff, Cyano, Halogen, gegebenenfalls mit Halogen oder CN substituiertes C₁-C₄-Alkyl oder für gegebenenfalls mit Halogen substituiertes C₁-C₄-Alkoxy steht,
durch Halogenierung von Verbindungen der Formel (II) 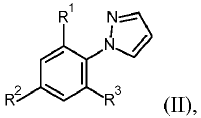 worin R¹, R² und R³ wie oben stehend definiert sind,
mit einer organischen halogenierenden Verbindung unter Zusatz von ≥ 0,0001 Äquivalenten und < 0,3 Äquivalenten, bezogen auf die gesamte eingesetzte Stoffmenge an Verbindung der Formel (II), wenigstens einer Säure, ausgewählt aus Mineralsäuren, Sulfonsäuren, Carbonsäuren und Lewis-Säuren.

2. Verfahren gemäß Anspruch 1, **dadurch gekennzeichnet, dass** X für Chlor, Brom oder Iod, bevorzugt für Brom oder Iod steht.

3. Verfahren gemäß einem der Ansprüche 1 bis 2, **dadurch gekennzeichnet, dass** die halogenierende Verbindung ausgewählt ist aus den *N*-Halogensuccinimiden, den 1,3-Dihalogen-5-5-dimethylhydantoinen oder den Halogencyanursäuren.

4. Verfahren gemäß einem der Ansprüche 1 bis 3, **dadurch gekennzeichnet, dass** die halogenierende Verbindung ausgewählt ist aus *N*-Bromsuccinimid (NBS), *N*-Iodsuccinimid (NIS), 1,3-Dibrom-5-5-dimethylhydantoin (DBDMH), 1,3-Diiod-5-5-dimethylhydantoin (DIDMH), 1,3,5-Tribrom-1,3,5-triazin-2,4,6-trion oder 1,3-Dibrom-1,3,5-triazin-2,4,6-trion.

5. Verfahren gemäß einem der Ansprüche 1 bis 4, **dadurch gekennzeichnet, dass** die Säure in einem Anteil von ≥ 0,001 Äquivalenten und ≤ 0,15 Äquivalenten, bezogen auf die gesamte eingesetzte Stoffmenge an Verbindung (II), eingesetzt wird.

6. Verfahren gemäß einem der Ansprüche 1 bis 5, **dadurch gekennzeichnet, dass** die Carbonsäuren einen pks-Wert von ≤ 5 aufweisen.

7. Verfahren gemäß einem der Ansprüche 1 bis 6, **dadurch gekennzeichnet, dass** die Säure ausgewählt ist aus HF, HCl, HBr, HI, H₂SO₄, HNO₃ und H₃PO₄, gegebenenfalls substituierten Aryl- und Alkylsulfonsäuren, gegebenenfalls substituierte Alkyl- und Arylcarbonsäuren, gegebenenfalls substituierte Alkyl- und Aryl-dicarbonsäuren, wobei die Carbonsäuren einen pks-Wert von ≤ 5 aufweisen, und wasserfreien oder hydratisierten Fluorid-, Chlorid- oder Bromid-Salzen, Nitraten, Acetaten, Sulfaten oder Trifluormethansulfonaten (OTf) von Lithium oder der Erdalkalimetalle, der Metalle der Borgruppe und der Übergangsmetalle.

8. Verfahren gemäß einem der Ansprüche 1 bis 6, **dadurch gekennzeichnet, dass** die Säure ausgewählt ist aus HCl, HF, HNO₃, H₂SO₄, H₃PO₄, Methansulfonsäure, Trifluormethansulfonsäure, Benzolsulfonsäure, para-Toluolsulfonsäure, Essigsäure, Propionsäure, Trifluoressigsäure, Trichloressigsäure und den wasserfreien oder hydratisierten Salzen ausgewählt aus den Fluorid-, Chlorid- oder Bromid-Salzen, Nitrate oder Trifluormethansulfonate (OTf) der Metalle B oder Al, aus den Nitraten oder Trifluormethansulfonaten (OTf) der Erdalkalimetalle Mg oder Ca oder den Nitraten oder Trifluormethansulfonaten (OTf) der Übergangsmetalle Fe, Zn, Cu oder Sc.

9. Verfahren gemäß einem der Ansprüche 1 bis 6, **dadurch gekennzeichnet, dass** die Säure ausgewählt ist aus HNO₃, HF, H₂SO₄, Methansulfonsäure, para-Toluolsulfonsäure, Essigsäure, Trifluoressigsäure, Trichloressigsäure, Mg(NO₃)₂, Ca(NO₃)₂, Fe₂(NO₃)₃, Zn(NO₃)₂, Zn(OTf)₂, Cu(NO₃)₂, Sc(NO₃)₃, Ca(OTf)₂, Mg(OTf)₂, Cu(OTf)₂, BBr₃, BCl₃, BF₃^{∗}OEt₂, Al(NO₃)₃, Al(OTf)₃, Fe(OTf)₃, Cu(OTf)₂ und Sc(OTf)₃.

10. Verfahren gemäß einem der Ansprüche 1 bis 9, **dadurch gekennzeichnet, dass** die Reaktion in einem Temperaturbereich von -78 bis 200 °C, bevorzugt bei Temperaturen zwischen -20 bis 100 °C durchgeführt wird.

11. Verfahren gemäß einem der Ansprüche 1 bis 10, **dadurch gekennzeichnet, dass** R² für mit Halogen substituiertes C₁-C₄-Alkyl oder mit Halogen substituiertes C₁-C₄-Alkoxy steht.

12. Verfahren gemäß einem der Ansprüche 1 bis 11, **dadurch gekennzeichnet, dass** R² für mit Fluor substituiertes C₁-C₄-Alkyl oder mit Fluor substituiertes C₁-C₄-Alkoxy steht.

13. Verfahren gemäß einem der Ansprüche 1 bis 12, **dadurch gekennzeichnet, dass** R¹ und R³ jeweils unabhängig voneinander für einen Substituenten ausgewählt aus Wasserstoff, Cl, Br, F, C₁-C₃-alkyl, mit Halogen substituiertes C₁-C₃-alkyl, C₁-C₃-alkoxy oder mit Halogen substituiertes C₁-C₃-alkoxy stehen.

14. Verfahren gemäß einem der Ansprüche 1 bis 13, **dadurch gekennzeichnet, dass** R¹ und R³ nicht gleichzeitig in einer Verbindung für Wasserstoff stehen.

15. Verfahren gemäß einem der Ansprüche 1 bis 14, **dadurch gekennzeichnet, dass**
R¹ für Halogen oder C₁-C₃-alkyl,
R² für mit Fluor substituiertes C₁-C₄-Alkyl oder mit Fluor substituiertes C₁-C₄-Alkoxy und
R³ für Halogen, C₁-C₃-alkyl oder mit Fluor substituiertes C₁-C₃-alkyl, C₁-C₃-alkoxy oder mit Fluor substituiertes C₁-C₃-alkoxy steht.

## Claims

1. Process for preparing compounds of the formula (I) 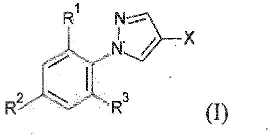 in which
X is halogen;
R¹ is hydrogen, cyano, halogen, C₁-C₄-alkyl optionally substituted by halogen or CN, or C₁-C₄-alkoxy optionally substituted by halogen,
R² is trifluoromethylsulfonyl, trifluoromethylsulfinyl, trifluoromethylsulfanyl, halogen, C₁-C₄-alkyl optionally substituted by halogen, or C₁-C₄-alkoxy optionally substituted by halogen and
R³ is hydrogen, cyano, halogen, C₁-C₄-alkyl optionally substituted by halogen or CN, or C₁-C₄-alkoxy optionally substituted by halogen,
by halogenating compounds of the formula (II) 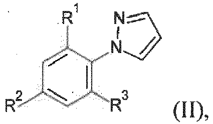 in which R¹, R² and R³ are as defined above,
with an organic halogenating compound with addition of ≥ 0.0001 equivalent and < 0.3 equivalent, based on the total molar amount of compound of the formula (II) used, of at least one acid selected from mineral acids, sulfonic acids, carboxylic acids and Lewis acids.

2. Process according to Claim 1, **characterized in that** X is chlorine, bromine or iodine, preferably bromine or iodine.

3. Process according to either of Claims 1 and 2, **characterized in that** the halogenating compound is selected from the N-halosuccinimides, the 1,3-dihalo-5,5-dimethylhydantoins or the halocyanuric acids.

4. Process according to any of Claims 1 to 3, **characterized in that** the halogenating compound is selected from *N*-bromosuccinimide (NBS), *N*-iodosuccinimide (NIS), 1,3-dibromo-5,5-dimethylhydantoin (DBDMH), 1,3-diiodo-5,5-dimethylhydantoin, (DIDMH), 1,3,5-tribromo-1,3,5-triazine-2,4,6-trione or 1,3-dibromo-1,3,5-triazine-2,4,6-trione.

5. Process according to any of Claims 1 to 4, **characterized in that** the acid is used in a proportion of ≥ 0.001 equivalent and ≤ 0.15 equivalent, based on the total molar amount of compound (II) used.

6. Process according to any of Claims 1 to 5, **characterized in that** the carboxylic acids have a pKa of ≤ 5.

7. Process according to any of Claims 1 to 6, **characterized in that** the acid is selected from HF, HCl, HBr, HI, H₂SO₄, HNO₃ and H₃PO₄, optionally substituted arylsulfonic and alkylsulfonic acids, optionally substituted alkylcarboxylic and arylcarboxylic acids, optionally substituted alkyldicarboxylic and aryldicarboxylic acids, where the carboxylic acids have a pKa of ≤ 5, and anhydrous or hydrated fluoride, chloride or bromide salts, nitrates, acetates, sulfates or trifluoromethanesulfonates (OTf) of lithium or of the alkaline earth metals, of the boron-group metals and of the transition metals.

8. Process according to any of Claims 1 to 6, **characterized in that** the acid is selected from HCl, HF, HNO₃, H₂SO₄, H₃PO₄, methanesulfonic acid, trifluoromethanesulfonic acid, benzenesulfonic acid, para-toluenesulfonic acid, acetic acid, propionic acid, trifluoroacetic acid, trichloroacetic acid and anhydrous or hydrated salts selected from the fluoride, chloride or bromide salts, nitrates or trifluoromethanesulfonates (OTf) of the metals B or Al, from the nitrates or trifluoromethanesulfonates (OTf) of the alkaline earth metals Mg or Ca, or the nitrates or trifluoromethanesulfonates (OTf) of the transition metals Fe, Zn, Cu or Sc.

9. Process according to any of Claims 1 to 6, **characterized in that** the acid is selected from HNO₃, HF, H₂SO₄, methanesulfonic acid, para-toluenesulfonic acid, acetic acid, trifluoroacetic acid, trichloroacetic acid, Mg(NO₃)₂, Ca(NO₃)₂, Fe₂(NO₃)₃, Zn(NO₃)₂, Zn(OTf)₂, Cu(NO₃)₂, Sc(NO₃)₃, Ca(OTf)₂, Mg(OTf)₂, Cu(OTf)₂, BBr₃, BCl₃, BF₃^{∗}OEt₂, Al(NO₃)₃, Al(OTf)₃, Fe(OTf)₃, Cu(OTf)₂ and Sc(OTf)₃.

10. Process according to any of Claims 1 to 9, **characterized in that** the reaction is conducted in a temperature range of -78 to 200°C, preferably at temperatures from -20 to 100°C.

11. Process according to any of Claims 1 to 10, **characterized in that** R² is halogen-substituted C₁-C₄-alkyl or halogen-substituted C₁-C₄-alkoxy.

12. Process according to any of Claims 1 to 11, **characterized in that** R² is fluorine-substituted C₁-C₄-alkyl or fluorine-substituted C₁-C₄-alkoxy.

13. Process according to any of Claims 1 to 12, **characterized in that** R¹ and R³ in each case independently of one another are a substituent selected from hydrogen, Cl, Br, F, C₁-C₃-alkyl, halogen-substituted C₁-C₃-alkyl, C₁-C₃-alkoxy or halogen-substituted C₁-C₃-alkoxy.

14. Process according to any of Claims 1 to 13, **characterized in that** R¹ and R³ are not simultaneously hydrogen in any compound.

15. Process according to any of Claims 1 to 14, **characterized in that**
R¹ is halogen or (C₁-C₃)-alkyl,
R² is fluorine-substituted C₁-C₄-alkyl or fluorine-substituted C₁-C₄-alkoxy and
R³ is halogen, C₁-C₃-alkyl or fluorine-substituted C₁-C₃-alkyl, C₁-C₃-alkoxy or fluorine-substituted C₁-C₃-alkoxy.

## Revendications

1. Procédé de préparation de composés de formule (I) 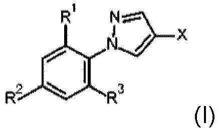 dans laquelle
X représente un halogène ;
R¹ représente un hydrogène, un cyano, un halogène, un alkyle en C₁-C₄, le cas échéant substitué par un ou des halogène(s) ou CN, ou un alcoxy en C₁-C₄, le cas échéant substitué par un ou des halogène(s),
R² représente un trifluorméthylsulfonyle, un trifluorméthylsulfinyle, un trifluorméthylsulfanyle, un halogène, un alkyle en C₁-C₄, le cas échéant substitué par un ou des halogène(s) ou un alcoxy en C₁-C₄, le cas échéant substitué par un ou des halogène(s), et
R³ représente un hydrogène, un cyano, un halogène, un alkyle en C₁-C₄, le cas échéant substitué par un ou des halogène(s) ou un CN, ou un alcoxy en C₁-C₄, le cas échéant substitué par un ou des halogène(s),
par halogénation de composés de formule (II) 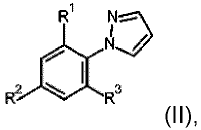
dans laquelle R¹, R² et R³ sont définis comme ci-dessus,
avec un composé organique d'halogénation en présence de ≥ 0,0001 équivalent à < 0,3 équivalent, sur base de la quantité totale de composé de la formule (II), d'au moins un acide, choisi parmi les acides minéraux, les acides sulfoniques, les acides carboxyliques et les acides de Lewis.

2. Procédé selon la revendication 1, **caractérisé en ce que** X représente le chlore, le brome ou l'iode, de préférence le brome ou l'iode.

3. Procédé selon l'une des revendications 1 à 2, **caractérisé en ce que** le composé d'halogénation est choisi parmi les *N*-halogénosuccinimides, les 1,3-dihalogéno-5,5-diméthylhydantoïnes ou les acides halogénocyanuriques.

4. Procédé selon l'une des revendications 1 à 3, **caractérisé en ce que** le composé d'halogénation est choisi parmi le *N*-bromosuccinimide (NBS), le N-iodoosuccinimide (NIS), la 1,3-dibromo-5,5-diméthylhydantoïne (DBDMH), la 1,3-diiodo-5,5-diméthylhydantoïne (DIDMH), la 1,3,5-tribromo-1,3,5-triazin-2,4,6-trione ou la 1,3-dibromo-1,3,5-triazin-2,4,6-trione.

5. Procédé selon l'une des revendications 1 à 4, **caractérisé en ce que** l'acide est utilisé en une quantité allant de ≥ 0,001 équivalent à ≤ 0,15 équivalent, sur la base de la quantité totale de composé (II).

6. Procédé selon l'une des revendications 1 à 5, **caractérisé en ce que** les acides carboxyliques présentent un pKs ≤ 5.

7. Procédé selon l'une des revendications 1 à 6, **caractérisé en ce que** l'acide est choisi parmi HF, HCl, HBr, Hl, H₂SO₄, HNO₃ et H₃PO₄, des acides aryl- et alkylsulfoniques le cas échéant substitués, des acides alkyl- et arylcarboxyliques le cas échéant substitués, des acides alkyl- et aryldicarboxyliques le cas échéant substitués, les acides carboxyliques présentant un pKs ≤ 5, et des sels de fluorure, de chlorure ou de bromure anhydres ou hydratés, des nitrates, des acétates, des sulfates ou des trifluorméthansulfonates (OTf) de lithium ou des métaux alcalino-terreux, des métaux du groupe bore ou des métaux de transition.

8. Procédé selon l'une des revendications 1 à 6, **caractérisé en ce que** l'acide est choisi parmi HCl, HF, HNO₃, H₂SO₄, H₃PO₄, l'acide méthanesulfonique, l'acide trifluorméthansulfonique, l'acide benzènesulfonique, l'acide para-toluènesulfonique, l'acide acétique, l'acide propionique, l'acide trifluoroacétique, l'acide trichloroacétique, et les sels anhydres ou hydratés choisis parmi les sels fluorure, chlorure ou bromure, les nitrates ou les trifluorméthansulfonates (OTf) des métaux B ou Al, les nitrates ou les trifluorméthansulfonates (OTf) des métaux alcalino-terreux Mg ou Ca ou les nitrates ou les trifluorméthansulfonates (OTf) des métaux de transition Fe, Zn, Cu ou Sc.

9. Procédé selon l'une des revendications 1 à 6, **caractérisé en ce que** l'acide est choisi parmi HNO₃, HF, H₂SO₄, l'acide méthanesulfonique, l'acide para-toluènesulfonique, l'acide acétique, l'acide trifluoroacétique, l'acide trichloroacétique, Mg(NO₃)₂, Ca(NO₃)₂, Fe₂(NO₃)₃, Zn(NO₃)₂, Zn(OTf)₂, Cu(NO₃)₂, Sc(NO₃)₃, Ca(OTf)₂, Mg(OTf)₂, Cu(OTf)₂, BBr₃, BCl₃, BF₃^{∗}OEt₂, Al(NO₃)₃, Al(OTf)₃, Fe(OTf)₃, Cu(OTf)₂ et Sc(OTf)₃.

10. Procédé selon l'une des revendications 1 à 9, **caractérisé en ce que** la réaction est réalisée à une température située dans l'intervalle allant de -78 à 200 °C, de préférence à une température allant de -20 à 100 °C.

11. Procédé selon l'une des revendications 1 à 10, **caractérisé en ce que** R² représente un alkyle en C₁-C₄ substitué par un ou des halogène(s) ou un alcoxy en C₁-C₄ substitué par un ou des halogène(s).

12. Procédé selon l'une des revendications 1 à 11, **caractérisé en ce que** R² représente un alkyle en C₁-C₄ substitué par un ou des fluor(s) ou un alcoxy en C₁-C₄ substitué par un ou des fluor(s).

13. Procédé selon l'une des revendications 1 à 12, **caractérisé en ce que** R¹ et R³ représentent indépendamment un substituant choisi parmi un hydrogène, Cl, Br, F, un alkyle en C₁-C₃, un alkyle en C₁-C₃ substitué par un ou des halogène(s), un alcoxy en C₁-C₃, ou un alcoxy en C₁-C₃ substitué par un ou des halogène(s).

14. Procédé selon l'une des revendications 1 à 13, **caractérisé en ce que** R¹ et R³ ne représentant pas en même temps un hydrogène dans un composé.

15. Procédé selon l'une des revendications 1 à 14, **caractérisé en ce que**
R¹ représente un halogène ou un alkyle en C₁-C₃,
R² représente un alkyle en C₁-C₄ substitué par un ou des fluor(s) ou un alcoxy en C₁-C₄ substitué par un ou des fluor(s), et
R³ représente un halogène, un alkyle en C₁-C₃ ou un alkyle en C₁-C₃ substitué par un ou des fluor(s), un alcoxy en C₁-C₃ ou un alcoxy en C₁-C₃ substitué par un ou des fluor(s).
